# EUROPEAN PATENT SPECIFICATION

(11) **EP 3 320 903 B1**
(45) Date of publication and mention of the grant of the patent: **19.05.2021**
(21) Application number: 16821693.5
(22) Date of filing: 08.07.2016
(51) Int. Cl.: A61K 31/4422, A61K 31/41, A61K 31/505, A61K 9/20, A61K 9/24

(54) **PHARMACEUTICAL COMPOSITION CONTAINING AMLODIPINE, VALSARTAN, AND ROSUVASTATIN**
PHARMAZEUTISCHE ZUSAMMENSETZUNG MIT AMLODIPIN, VALSARTAN UND ROSUVASTATIN
COMPOSITION PHARMACEUTIQUE CONTENANT L'AMLODIPINE, LE VALSARTAN ET LA ROSUVASTATINE

(30) Priority: 08.07.2015 KR 20150097373
(43) Date of publication of application: 16.05.2018
(73) Proprietor: HK inno.N Corporation, Seoul 04551 (KR)
(72) Inventor: JUNG, Yoon Seok, Incheon 21420 (KR); LYU, Chun Seon, Yongin-si Gyeonggi-do 17072 (KR); OH, Tack Oon, Gwangju-si Gyeonggi-do 12771 (KR); JEON, Eun Kyung, Hwaseong-si Gyeonggi-do 18446 (KR); HAN, Sung Kyun, Hwaseong-si Gyeonggi-do 18482 (KR); RYU, Chae Young, Anyang-si Gyeonggi-do 13929 (KR)
(74) Representative: Ruscoe, Peter James
(86) International application number: PCT/KR2016/007464
(87) International publication number: WO 2017/007287

(56) References cited:
- WO-A1-2009/084003
- WO-A1-2015/051771
- WO-A2-2011/102702
- CN-A- 101 637 609
- KR-A- 20120 011 158
- KR-A- 20120 099 320
- KR-B1- 101 207 618
- DATABASE BIOSIS [Online] BIOSCIENCES INFORMATION SERVICE, PHILADELPHIA, PA, US; November 2013 (2013-11), CESKA RICHARD ET AL: "Complex Approach in the Decrease of Cardiovascular Risk in Daily Practice, the Varo Study", XP55552585, Database accession no. PREV201400365293

## Description

### [Technical Field]

The present disclosure relates to a pharmaceutical composition comprising a first composition which comprises amlodipine or a pharmaceutically acceptable salt thereof and valsartan or a pharmaceutically acceptable salt thereof; and a second composition which comprises rosuvastatin or a pharmaceutically acceptable salt thereof, and a preparation method thereof.

### [Background Art]

Hypertension is the most common cardiovascular disease, and damages blood vessels of the kidney, heart, and brain as blood pressure continually rises and elevates, thereby increasing the incidence of renal failure, coronary artery disease, heart failure, and stroke. Hypertension is divided into essential hypertension and secondary hypertension. Essential hypertension refers to high blood pressure of an unknown cause, that is, high blood pressure without a particular causative disease. Essential hypertension accounts for most (approximately 95%) of all hypertension cases, and for those in their 40s or older, most cases are related to essential hypertension. As there is no disease that causes essential hypertension, it is difficult to clearly investigate the cause thereof; however, genetic predisposition, salty eating habits, obesity, old age, stress, and excessive smoking and drinking can be problematic. Secondary hypertension refers to high blood pressure of a particular disease which secondarily increases blood pressure. Secondary hypertension accounts for 5% of all hypertension cases, and cases thereof in relatively younger people are related thereto. Nephritis, endocrine system abnormality, and pregnancy toxemia are primary causes, and blood pressure naturally lowers when the causative disease is treated.

Antihypertensive agents, which lower blood pressure, are broadly classified into three types-diuretics, antiadrenergic agents, and vasodilators-according to major regulatory sites or mechanisms, and are divided more specifically according to the sites on which each drug acts. Diuretics are drugs which excrete water and salt from the body by increasing an amount of urine. They lower blood pressure by reducing the amounts of water and salt in the body. The sympathetic nervous system increases the number of contractions of the heart and strengthens the contractions, and also contracts the blood vessels, and sympathetic inhibitors are drugs that suppress the activities of the sympathetic nervous system, thereby lowering blood pressure. Sympatholytic agents include alpha blockers, which suppress the sympathetic nervous system that contracts blood vessels, beta blockers, which suppress the sympathetic nervous system that contracts the heart, and centrally acting sympatholytic agents, which act on the brain. Vasodilators are drugs which lower blood pressure by dilating blood vessels, and there are several types of vasodilators, such as ACE inhibitors, which suppress production of the vasoconstrictor angiotensin II, and angiotensin II receptor antagonists, which block angiotensin II activities. Additionally, as blood vessels contract and blood pressure elevates when intracellular calcium ion concentration increases, calcium channel blockers, which block the entry of the calcium ions and thereby lower blood pressure, are also a vasodilator.

It is important to treat hypertension by preventing life-threatening complications of coronary artery diseases such as stroke, heart failure, myocardial infarction, *etc.;* and cardiovascular complications such as renal failure by maintaining blood pressure within a normal range, rather than treating the blood pressure itself. Therefore, it is important to steadily and patiently control blood pressure. Further, antihypertensive medications require life-long administration, and thus, a treatment drug should be carefully selected. Therefore, for continued treatment, it is necessary to obtain more excellent prophylactic and therapeutic effects by co-administering drugs having different mechanisms and reduce risks of side effects that can be caused by the long-term administration of a single drug by reducing an amount of its intake through the co-administration of drugs, rather than selecting a single drug. The Seventh Report of the Joint National Committee on Prevention, Detection, Evaluation, and Treatment of High Blood Pressure (JNC7) recommends co-administration of drugs having different mechanisms in a case where there is no sufficient effect of blood pressure control by a single drug administration.

Meanwhile, hyperlipidemia means abnormally elevated levels of lipids in the blood, such as cholesterol, triglycerides, *etc.* In particular, hypercholesterolemia, by inducing coronary thrombosis, induces arteriosclerosis, in which an artery wall thickens as a result of accumulation of lipids, and causes ischemic heart disease, angina, and myocardial infarction by decreasing blood flow. Likewise, hyperlipidemia and arteriosclerosis are closely related, and thus, arteriosclerosis can be prevented by treating hyperlipidemia.

As HMG-CoA reductase inhibitors inhibit mevalonate production to thereby interrupt cholesterol biosynthesis and show an effect of lowering levels of total cholesterol and LDL-cholesterol, they have been used in the treatment of hyperlipidemia (see Grundi S. M., NEngl J Med, 319(1): 24-32, 25-26, 31(1998)).

Hypertension is often prevalent in hyperlipidemia, and they are considered as major risk factors for cardiovascular diseases, ultimately leading to adverse cardiac symptoms. Such risk factors originate from potentially common mechanisms. Accordingly, it is advantageous for patients to receive a single prescription of a drug capable of treating all of said diseases; however, when cardiovascular disease patients co-administer an angiotensin II receptor antagonist and an HMG-CoA reductase inhibitor, not only are hypertension and hyperlipidemia treated, but also the function of endothelial cells as a blood vessel protective membrane is improved, as well as increased sensitivity to insulin, thereby showing therapeutic effects in diabetes (see Ceriello A, Assaloni R, Da Ros R, Maier A, Piconi L, Quagliaro L, et al., Circulation, 111: 2518-2524, May 2005; and Koh KK, Quon MJ, Han SH, et al., Circulation, 110: 3687-3692, Dec 2004). Meanwhile, it is known that a large synergistic effect can be obtained by administering a calcium channel blocker, a therapeutic agent for hypertension, together with a lipid-lowering agent to treat arteriosclerosis (see Kramsch et al., Journal of Human Hypertension, Suppl.1, 53-59, 1995). It is also known that the calcium channel blocker can have an advantageous effect in the treatment of early atherosclerotic lesions (see Lichtlen P.R. et al., Lancet, 335, 1109-1139, 1990 and Waters D et al., Circulation, 82, 1940-1953, 1990). Combinations of a compound having a hyperlipidemia therapeutic activity and an antihypertensive drug are disclosed in various other references (WO 95/26188, WO 97/37688, WO 99/11260, WO 00/45818, WO 04/062729, WO 06/040085, *etc.).*

In this regard, the present disclosure is to obtain the synergistic effect as described above and mitigate side effects, as well as to improve administration compliance, by providing a combination formulation including an angiotensin II receptor antagonist, a therapeutic agent for hypertension such as a calcium channel blocker, and a lipid-lowering agent such as an HMG-CoA reductase inhibitor as major ingredients WO 2011/102702 A2 discloses a Valsartan comprising tablet core which is compression coated with a granulate comprising at least one further active substance selected from calcium channel blockers such as amlodipine or its salts, and/or diuretic such as hydrochlorothiazide and/or indapamide, and/or cholesterol lowering agent such as HMG-CoA reductase inhibitors, such as rosuvastatin, simvastatin, lovastatin, atorvastatin, fibrates, bile acid sequestrants and/or nicotinic acid. CN 101 637 609 A discloses pharmaceutical compositions comprising amlodipine, an angiotensin II receptor antagonist and a statin. R. Ceska, Scientific Sessions and Resuscitation Science Symposium of the American-Heart-Association (Dallas, TX, USA; November 2013) discloses the VARO clinical study (Valsartan Amlodipin and ROsuvastatin for global cardiovascular risk).

According to WO 2006/059217, amlodipine, one of the calcium channel blockers, is highly hygroscopic and absorbs moisture, leading to degradation. One of the major routes of degradation is via the catalytic oxidative process, which is pH-dependent. Pharmaceutical Development and Technology (Vol. 9, No. 1, pp. 15-24, 2004) describes that mixtures of lactose, a basic excipient, and water induce some instability on amlodipine besylate due to occurrence of the Maillard reaction between primary amines and lactose.

Being an unstable compound, amlodipine thus requires well-directed stability approaches with regard to selection of types of active ingredients and excipients and contents thereof to formulate pharmaceutical compositions with reasonable stability.

Additionally, valsartan, one of the angiotensin II receptor antagonists, can be used for the purpose of the present disclosure in its free form as well as in any suitable salt form. In the United States, an initial dose of valsartan as a therapeutic agent for hypertension is 80 mg to 160 mg daily administration, and the maximum daily dose is 320 mg. In Korea, valsartan is prescribed for patients with cardiac insufficiency or post myocardial infarction syndrome in a dose of 160 mg twice a day. However, if blood pressure of the patient does not reach a desired value with the dose of 160 mg twice a day, valsartan is replaced with other drugs. Valsartan is conventionally used in a dose of about 40 mg to 320 mg, preferably about 80 mg to 320 mg, most preferably about 80 mg to 160 mg in a tablet.

Prior art related to valsartan has difficulty in tableting as the density of most raw materials is low, and thus requires formation of a coprimate through capsulation or compaction of dry ground ingredients. Such complicated process is accompanied by difficulty in validation of a preparation method. In order to solve the difficulty, there have been studies on pellet formulations which use a mobile phase coating device, but the formulation has problems of increased preparation cost and inconvenience of administration due to an increased capsule volume.

Formulations containing a rosuvastatin calcium salt have a problem in that they degrade easily under a particular condition. In this regard, UK Patent No. 2262229 discloses pharmaceutical formulations of certain 7-substituted-3,5-dihydroxy-6-heptenoic acid salts, which are HMG-CoA reductase inhibitors, and also discloses that the formulation requires an alkaline medium (e.g., carbonate salts or bicarbonate salts) capable of imparting a pH of at least 8 to an aqueous solution or dispersion of the composition. Additionally, Determination of Rosuvastatin in the presence of Its Degradation Products by a Stability-Indicating LC Method (Journal of AOAC International Vol. 88, No. 4, 2005) reports that rosuvastatin calcium salts are easily degraded by an acidic condition of pH 5 or lower, oxidation, light, and thermal stress. As for a formulation, a granular product with a larger area exposed to the environment is less stable than an uncoated tablet, and a film-coated tablet is more stable than an uncoated tablet.

Accordingly, it is important to formulate a pharmaceutical composition of rosuvastatin calcium salts which are stable for a long period of time. Such composition has a reasonable flow rate and thus can be easily processed in an oral formulation. In the case of a tablet for oral administration, it is preferable that the tablet have reasonable disintegrability and solubility.

Developing the above combination formulation containing amlodipine, valsartan, and rosuvastatin by simply mixing may cause problems due to difference in unique physical properties of the active ingredients, *i.e.,* amlodipine, valsartan, and rosuvastatin.

The first problem is caused by difference in pKa values of the ingredients. The pKa values of amlodipine, valsartan, and rosuvastatin are about 8.6, 3.9, and 4.6, respectively. Such difference in the pKa values would differently influence the solubility of each ingredient.

The second problem is gelation of valsartan. Valsartan, due to its low solubility, undergoes gelation at pH 4.0 or lower, leading to extremely slow dissolution. Such slow dissolution causes valsartan to be absorbed in the small intestine. Additionally, by the gelation of valsartan, dissolution of amlodipine and rosuvastatin may be slowed.

Lastly, in the case of a combination formulation in which amlodipine, valsartan, and rosuvastatin are simultaneously mixed, stability decreases due to interactions between major ingredients or those between major ingredients and excipients.

In this regard, the present inventors made extensive efforts to develop a formulation capable of obtaining optimized stability and dissolution rate, thereby completing the present disclosure.

### [Disclosure]

### [Technical Problem]

An object of the present disclosure is to provide a novel composition which has improved therapeutic effects and medication compliance and mitigates side effects, by co-administering at least two types of therapeutic agents for hypertension and lipid-lowering agents for their synergistic reactions.

Additionally, an object of the present disclosure is to provide a pharmaceutical composition comprising a first composition which comprises amlodipine or a pharmaceutically acceptable salt thereof and valsartan or a pharmaceutically acceptable salt thereof; and a second composition which comprises rosuvastatin or a pharmaceutically acceptable salt thereof, which has excellent stability and dissolution rate of active ingredients; and a preparation method thereof.

Additionally, another object of the present disclosure is to provide a pharmaceutical composition, as the composition comprising amlodipine or a pharmaceutically acceptable salt thereof, valsartan or a pharmaceutically acceptable salt thereof, and rosuvastatin or a pharmaceutically acceptable salt thereof, further comprising any one selected from the group consisting of magnesium aluminosilicate and low-substituted hydroxypropyl cellulose and any one selected from the group consisting of anhydrous dibasic calcium phosphate and calcium carbonate; and a preparation method thereof.

### [Technical Solution]

In order to solve the problem, the present disclosure provides a pharmaceutical composition, comprising a first composition which comprises amlodipine or a pharmaceutically acceptable salt thereof and valsartan or a pharmaceutically acceptable salt thereof; and a second composition which comprises rosuvastatin or a pharmaceutically acceptable salt thereof The compositions according to the invention are defined in the appended claims.

As disclosed in US Patent Nos. 4,572,909 and 4,879,303, amlodipine is a calcium ion channel blocker developed for treatment of hypertension and other medication indicants. The chemical name thereof is 3-ethyl-5-methyl-(+-)-2-[(2-aminoethoxy)methyl]-4-(2-chlorophenyl)-1,4-dihydro-6-methylpyridine-3,5-dicarboxylate. Valsartan is an angiotensin II receptor antagonist having activities of vasoconstrictor and sodium retention, and lowers blood pressure through vasodilation. The chemical name thereof is ((*S*)-*N*-valery-*N*-{[2'-(1*H*-tetrazol-5-yl)-biphenyl-4-yl]-methyl}-valine). Rosuvastatin is a HMG-CoA reductase inhibitor, and is used in treatment of hypercholesterolemia, hyperlipoproteinemia, and atherosclerosis. The chemical name thereof is (3*R*,5*S*,6*E*)-7-[4-(4-fluorophenyl)-2-(*N-*methylmethanesulfonamido)-6-(propan-2-yl)pyrimidin-5-yl]-3,5-dihydroxyhepten-6-oic acid).

The amlodipine, valsartan, and rosuvastatin, which are isolated or obtained from natural sources, can be prepared by chemical modification or chemical synthesis known in the art easily by one of ordinary skill in the art. In addition, commercially available products can be purchased and used.

Preferably, the pharmaceutical composition comprises 5 mg to 10 mg of amlodipine, 80 mg to 160 mg of valsartan, or 5 mg to 20 mg of rosuvastatin per unit dosage form.

The present disclosure is a pharmaceutical composition of a combination formulation comprising all three major ingredients, and has the following advantages in co-administration thereof.

First, an effect of lowering blood pressure is improved by co-administering different drugs having different mechanisms to simultaneously act on. Further, by an effect of a drug in suppressing homeostatic compensation of another drug, blood pressure is lowered more effectively. For example, reflex tachycardia and retention of saline and water that can be caused by use of a vasodilator can be controlled by a beta blocker and a diuretic, respectively, and thus, the effect of lowering blood pressure can be improved.

Second, combination therapy can have the sufficient blood pressure-lowering effect even with a low dose, and thus can mitigate dose-dependent side effects. Additionally, by blocking side effects caused by pharmacological actions, side effects can be reduced.

Third, as hypertension requires lifelong control but has almost no subjective symptoms, it is important to maintain patient compliance with medication. Compliance can be improved by combination therapy, which has reduced rates of side effects and can further improve quality of life.

As used herein, the term "pharmaceutically acceptable salt" refers to a formulation which does not damage biological activities and properties of the administered amlodipine, valsartan, and rosuvastatin. The pharmaceutically acceptable salt is an acid *(e.g.,* an inorganic acid such as hydrochloric acid, sulfuric acid, nitric acid, phosphoric acid, hydrobromic acid, hydroiodic acid, *etc.;* an organic carboxylic acid such as tartaric acid, formic acid, citric acid, acetic acid, trichloroacetic acid, trifluoroacetic acid, gluconic acid, benzoic acid, lactic acid, fumaric acid, maleic acid, salicylic acid, *etc.;* a sulfonic acid such as methanesulfonic acid, ethanesulfonic acid, benzenesulfonic acid, *p*-toluenesulfonic acid, *etc.; etc.)* which forms a non-toxic acid addition salt containing a pharmaceutically acceptable anion. For example, the pharmaceutically acceptable carboxylic acid salts include metal salts or alkaline earth metal salts formed by lithium, sodium, potassium, calcium, magnesium, *etc.;* amino acid salts such as lysine, arginine, guanidine, *etc.;* organic salts such as dicyclohexylamine, *N*-methyl-D-glucamine, tris(hydroxymethyl)methylamine, diethanolamine, choline, triethylamine, *etc.; etc.* In the case of amlodipine, a commercially available besylic acid salt is preferable.

Among the pharmaceutical compositions of the present disclosure, the "first composition" includes amlodipine or a pharmaceutically acceptable salt thereof and valsartan or a pharmaceutically acceptable salt thereof, and the "second composition" includes rosuvastatin or a pharmaceutically acceptable salt thereof. According to an exemplary embodiment of the present disclosure, separating amlodipine and valsartan as the first composition and rosuvastatin as the second composition increases stability and has an excellent pattern of dissolution rate. There is no particular meaning in the order of the first and second compositions, and such order is merely to distinguish the two compositions.

The pharmaceutical composition of the present disclosure can be prepared in various formulations; for example, tablets such as uncoated tablets, film-coated tablets, single-layered tablets, double-layered tablets, multi-layer tablets, or core tablets; powders; granules; capsules; *etc.* The pharmaceutical composition of the present disclosure can preferably be formulated in a single-layered tablet or double-layered tablet.

According to an exemplary embodiment of the present disclosure, single-layered tablets show excellent stability and a dissolution rate similar to double-layered tablets, and thus can be prepared by a simple preparation process at a low cost.

Among the pharmaceutical compositions of the present disclosure, the first composition may be provided in the form of a granule. The granules, which are used in tableting, can be obtained by a granulation process wherein active components are mixed with additives, and sieved, and then mixed with a binding solution in purified water by a high-speed mixer, and dried.

The second composition may be provided in the form of a granule or may be mixed with the granulated first composition, and directly compressed to form a tablet.

Meanwhile, various additives, that is, excipients, disintegrating agents, or binding agents, can be added to formulate the pharmaceutical composition into a tablet. The excipients, disintegrating agents, or binding agents are not particularly limited, but starch or microcrystalline cellulose can be used as the excipient. A calcium salt of carboxymethyl cellulose, crospovidone, croscarmellose sodium, *etc.* can be used as the disintegrating agent. Additionally, as the binding agent, hydroxypropyl cellulose or polyvinylpyrrolidone can be used.

In particular, as a major excipient, alumina magnesium metasilicate, low-substituted hydroxypropyl cellulose, or a mixture thereof may be included. According to an exemplary embodiment of the present disclosure, alumina magnesium metasilicate, low-substituted hydroxypropyl cellulose, or a mixture thereof is the major excipient that affects the dissolution rate of major ingredients. It is preferable that the alumina magnesium metasilicate, low-substituted hydroxypropyl cellulose, or mixture thereof be comprised at 3 wt% to 15 wt% based on the total weight of the pharmaceutical composition.

Additionally, as a major excipient, anhydrous dibasic calcium phosphate, calcium carbonate, or a mixture thereof can be included. According to an exemplary embodiment of the present disclosure, anhydrous dibasic calcium phosphate, calcium carbonate, or a mixture thereof is the major excipient that affects the stability of major ingredients. It is preferable that the anhydrous dibasic calcium phosphate, calcium carbonate, or mixture thereof be comprised at 3 wt% to 5 wt% based on the total weight of the pharmaceutical composition.

According to an exemplary embodiment of the present disclosure, a pharmaceutical composition is provided as a double-layered tablet including the first composition as the first layer and the second composition as the second layer. There is no particular meaning in the order of the first and second compositions, but it is merely to distinguish the two compositions.

### [Advantageous Effects]

The combination formulation according to the present disclosure, comprising amlodipine, valsartan, and rosuvastatin, has excellent stability and a high dissolution rate of active ingredients, and by a synergistic combination of drugs having different mechanisms, mitigates side effects, and thus can be used more effectively in prevention and treatment of cardiovascular disease, hypertension, arteriosclerosis, hyperlipidemia, and a complex disease and improve medication compliance.

Additionally, the present disclosure is advantageous in that not only are interactions between main ingredients minimized to improve stability, but also the weight and size are less than those of a combination of commercially available drugs (Exforge Tab. and Crestor Tab.) corresponding to the above three ingredients.

### [Description of the Drawings]

Figs. 1a to 1c are graphs of the comparative dissolution test profile of the single-layered tablet of Experimental Example 2-1.
Figs. 2a to 2c are graphs of the comparative dissolution test profile of the double-layered tablet of Experimental Example 2-2.
Fig. 3 is an image of the single-layered tablet (Exforge Tab. 5/160 mg and Crestor Tab. 10 mg) prepared according to an exemplary embodiment of the present disclosure; and Exforge tablet and Crestor tablet used as the control drugs.
Figs. 4a to 4c are graphs of the comparative dissolution test profile of the single-layered tablet of Experimental Example 2-4.

### [Mode for Invention]

Hereinafter, the present invention will be described in more detail with reference to the following Examples. However, these Examples are for illustrative purposes only, and the invention is not intended to be limited by these Examples.

### Example 1 - A single-layered tablet formulated by preparing the granule of valsartan and amlodipine and mixing therewith the second composition comprising rosuvastatin

### 1) Preparation of granule of valsartan and amlodipine besylate

After mixing valsartan, amlodipine besylate, microcrystalline cellulose, alumina magnesium metasilicate, and croscarmellose sodium, the mixture was sieved through a No. 25 sieve. Then, hydroxypropyl cellulose was dissolved in an appropriate amount of purified water to prepare a binding solution. The mixture and the binding solution were mixed in a high-speed mixer and dried to be granulated.

### 2) Tableting a single-layered tablet

After mixing rosuvastatin calcium, anhydrous dibasic calcium phosphate, pregelatinized starch, microcrystalline cellulose, crospovidone, and croscarmellose sodium, the mixture was sieved through a No. 25 sieve. The mixture was further mixed with the above-prepared granule along with magnesuim stearate, and was then tableted.

The content of each ingredient is shown in Table 1 below.

**[Table 1]**

| Example 1 | Ingredients | Content (mg) |
|---|---|---|
| Granule | Valsartan | 160.00 |
| | Amlodipine besylate | 6.94 |
| | Microcrystalline cellulose | 90.66 |
| | Alumina magnesium metasilicate | 16.00 |
| | Croscarmellose sodium | 16.00 |
| | Hydroxypropyl cellulose | 9.60 |
| Further mixture | Rosuvastatin calcium | 10.40 |
| | Anhydrous dibasic calcium phosphate | 18.20 |
| | Pregelatinized starch | 15.30 |
| | Microcrystalline cellulose | 24.60 |
| | Crospovidone | 10.00 |
| | Croscarmellose sodium | 21.00 |
| | Magnesium stearate | 6.30 |
| Total weight | | 405.00 |

### Example 2 - A single-layered tablet formulated by preparing the granule of valsartan and amlodipine and mixing therewith rosuvastatin granule

### 1) Preparation of a granule of valsartan and amlodipine besylate

The granule was prepared in the same manner as Example 1, and polyvinyl pyrrolidone was used instead of hydroxypropyl cellulose.

### 2) Preparation of rosuvastatin granule

After mixing rosuvastatin calcium, anhydrous dibasic calcium phosphate, pregelatinized starch, microcrystalline cellulose, and crospovidone, the mixture was sieved through a No. 25 sieve. Using an appropriate amount of purified water as a binding solution, the mixture was mixed in a high-speed mixer and dried to be granulated.

### 3) Tableting a single-layered tablet

Croscarmellose sodium and magnesium stearate were further mixed in the granule of valsartan and amlodipine and the rosuvastatin granule to prepare a tablet using a table press machine.

The content of each ingredient is shown in Table 2 below.

**[Table 2]**

| Example 2 | Ingredients | Content (mg) |
|---|---|---|
| First granule | Valsartan | 160.00 |
| | Amlodipine besylate | 6.94 |
| | Microcrystalline cellulose | 90.66 |
| | Alumina magnesium metasilicate | 16.00 |
| | Croscarmellose sodium | 16.00 |
| | Polyvinyl pyrrolidone | 9.60 |
| Second granule | Rosuvastatin calcium | 10.40 |
| | Anhydrous dibasic calcium phosphate | 18.20 |
| | Pregelatinized starch | 15.30 |
| | Microcrystalline cellulose | 24.60 |
| | Crospovidone | 10.00 |
| Further mixture | Croscarmellose sodium | 21.00 |
| | Magnesium stearate | 6.30 |
| Total weight | | 405.00 |

### Example 3 - A single-layered tablet formulated by preparing the granule of valsartan and amlodipine and mixing therewith rosuvastatin granule

Using the same method as Example 2, a single-layered tablet having the ingredients and contents shown in Table 3 below was prepared.

**[Table 3]**

| Example 3 | Ingredients | Content (mg) |
|---|---|---|
| First granule | Valsartan | 160.00 |
| | Amlodipine besylate | 6.94 |
| | Microcrystalline cellulose | 90.66 |
| | Alumina magnesium metasilicate | 16.00 |
| | Croscarmellose sodium | 16.00 |
| | Hydroxypropyl cellulose | 9.60 |
| Second granule | Rosuvastatin calcium | 10.40 |
| | Anhydrous dibasic calcium phosphate | 18.20 |
| | Pregelatinized starch | 25.50 |
| | Microcrystalline cellulose | 25.50 |
| | Polyvinyl pyrrolidone | 2.00 |
| | Crospovidone | 6.40 |
| Further mixture | Crospovidone | 10.00 |
| | Magnesium stearate | 4.80 |
| Total weight | | 402.00 |

### Example 4 - A single-layered tablet formulated by preparing the granule of valsartan and amlodipine and mixing therewith the second composition containing rosuvastatin

Using the same method as Example 1, a single-layered tablet having the ingredients and contents shown in Table 4 below was formulated.

**[Table 4]**

| Example 4 | Ingredients | Content (mg) |
|---|---|---|
| Granule | Valsartan | 160.00 |
| | Amlodipine besylate | 6.94 |
| | Microcrystalline cellulose | 90.66 |
| | Alumina magnesium metasilicate | 16.00 |
| | Croscarmellose sodium | 16.00 |
| | Polyvinyl pyrrolidone | 9.60 |
| Further mixture | Rosuvastatin calcium | 10.40 |
| | Anhydrous dibasic calcium phosphate | 18.20 |
| | Pregelatinized starch | 25.40 |
| | Microcrystalline cellulose | 20.00 |
| | Crospovidone | 26.00 |
| | Magnesium stearate | 4.8 |
| Total weight | | 404.00 |

### Example 5 - A double-layered formulation comprising a first layer including a granule of valsartan and amlodipine and a second layer including a granule of rosuvastatin

### 1) Preparation of a granule of valsartan and amlodipine besylate

Using the same method as Example 2, a granule of valsartan and amlodipine besylate was prepared.

### 2) Preparation of rosuvastatin granule

Using the same method as Example 2, a rosuvastatin granule was prepared.

### 3) Preparation of double-layered tablet

The granules prepared in 1) and 2) were further mixed with magnesium stearate, respectively, and were then compressed using a double-layered tablet press machine to form a tablet having a first layer comprising the granule prepared in 1), and a second layer comprising the granule prepared in 2).

The content of each ingredient is shown in Table 5 below.

**[Table 5]**

| Example 5 | | Ingredients | Content (mg) |
|---|---|---|---|
| First layer | Granule | Valsartan | 160.00 |
| | | Amlodipine besylate | 6.94 |
| | | Microcrystalline cellulose | 90.66 |
| | | Alumina magnesium metasilicate | 16.00 |
| | | Croscarmellose sodium | 16.00 |
| | | Hydroxypropyl cellulose | 9.60 |
| | Further mixture | Croscarmellose sodium | 16.00 |
| | | Magnesium stearate | 4.80 |
| Second layer | Granule | Rosuvastatin calcium | 10.40 |
| | | Anhydrous dibasic calcium phosphate | 18.20 |
| | | Pregelatinized starch | 15.30 |
| | | Microcrystalline cellulose | 24.60 |
| | | Crospovidone | 10.00 |
| | Further mixture | Croscarmellose sodium | 5.00 |
| | | Magnesium stearate | 1.50 |
| Total weight | | | 473.26 |

### Example 6 - A double-layered formulation comprising first layer containing the granule of valsartan and amlodipine and the second layer containing rosuvastatin granule

Using the same method as Example 5, a double-layered tablet having the ingredients and contents shown in Table 6 below was prepared.

**[Table 6]**

| Example 6 | | Ingredients | Content (mg) |
|---|---|---|---|
| First layer | Granule | Valsartan | 160.00 |
| | | Amlodipine besylate | 6.94 |
| | | Microcrystalline cellulose | 90.66 |
| | | Croscarmellose sodium | 16.00 |
| | | Alumina magnesium metasilicate | 16.00 |
| | | Hydroxypropyl cellulose | 6.40 |
| | Further mixture | Croscarmellose sodium | 16.00 |
| | | Magnesium stearate | 2.40 |
| Second layer | Granule | Rosuvastatin calcium | 10.40 |
| | | Anhydrous dibasic calcium phosphate | 18.20 |
| | | Pregelatinized starch | 25.20 |
| | | Microcrystalline cellulose | 20.60 |
| | | Crospovidone | 6.40 |
| | | Hydroxypropyl cellulose | 2.00 |
| | Further mixture | Crospovidone | 10.00 |
| | | Croscarmellose sodium | 2.40 |
| Total weight | | | 412.80 |

### Example 7 - A single-layered tablet formulated by preparing the granule of valsartan and amlodipine and mixing therewith rosuvastatin granule

Using the same method as Example 2, a single-layered tablet having the ingredients and contents shown in Table 7 below was prepared.

**[Table 7]**

| Example 7 | Ingredients | Content (mg) |
|---|---|---|
| First granule | Valsartan | 160.00 |
| | Amlodipine besylate | 6.94 |
| | Microcrystalline cellulose | 90.66 |
| | Alumina magnesium metasilicate | 16.00 |
| | Croscarmellose sodium | 16.00 |
| | Hydroxypropyl cellulose | 9.60 |
| Second granule | Rosuvastatin calcium | 10.40 |
| | Anhydrous dibasic calcium phosphate | 18.20 |
| | Pregelatinized starch | 25.00 |
| | Microcrystalline cellulose | 20.00 |
| | Crospovidone | 6.40 |
| Further mixture | Crospovidone | 20.00 |
| | Magnesium stearate | 4.80 |
| Total weight | | 406.00 |

### Examples 8, 9, 10, and 14 - A single-layered tablet formulated by preparing the granule of valsartan and amlodipine and mixing therewith rosuvastatin granule

Using the same method as Example 2, a single-layered tablet having the ingredients and contents shown in Table 8 below was prepared.

**[Table 8]**

| | Ingredients | Example 8 | Example 9 | Example 10 | Example 14 |
|---|---|---|---|---|---|
| First granule | Valsartan | 80.00 | 160.00 | 160.00 | 160.00 |
| | Amlodipine besylate | 6.94 | 13.88 | 6.94 | 13.88 |
| | Microcrystalline cellulose | 45.33 | 90.70 | 65.33 | 100.72 |
| | Croscarmellose sodium | 8.00 | 20.00 | 20.00 | 19.00 |
| | Hydroxypropyl cellulose | 4.80 | 9.60 | 9.60 | 9.60 |
| | Alumina magnesium metasilicate | 8.00 | 27.00 | | 19.00 |
| | Low-substituted hydroxypropyl cellulose | | | 65.33 | |
| Second granule | Rosuvastatin calcium | 10.40 | 10.40 | 10.40 | 20.80 |
| | Pregelatinized starch | 25.00 | 25.00 | 25.00 | 20.00 |
| | Microcrystalline cellulose | 20.00 | 20.00 | 20.00 | 22.00 |
| | Crospovidone | 6.40 | 6.40 | 6.40 | 9.40 |
| | Hydroxypropyl cellulose | 2.00 | 2.00 | 2.00 | |
| | Anhydrous dibasic calcium phosphate | 18.20 | 18.20 | 18.20 | 20.80 |
| Further mixture | Crospovidone | 10.50 | 20.00 | 20.00 | 21.00 |
| | Magnesium stearate | 2.40 | 4.80 | 4.80 | 4.80 |
| Total weight | | 247.97 | 428.00 | 434.00 | 441.00 |

### Examples 11 to 13 - A single-layered tablet formulated by preparing the granule of valsartan and amlodipine and mixing therewith rosuvastatin granule

Using the same method as Example 2, a single-layered tablet having the ingredients and contents shown in Table 9 below was prepared.

**[Table 9]**

| | Ingredients | Example 11 | Example 12 | Example 13 |
|---|---|---|---|---|
| First granule | Valsartan | 160.00 | 160.00 | 160.00 |
| | Amlodipine besylate | 6.94 | 6.94 | 6.94 |
| | Microcrystalline cellulose | 90.66 | 90.66 | 90.66 |
| | Croscarmellose sodium | 16.00 | 16.00 | 16.00 |
| | Hydroxypropyl cellulose | 9.60 | 9.60 | 9.60 |
| | Alumina magnesium metasilicate | 16.00 | 16.00 | 16.00 |
| Second granule | Rosuvastatin calcium | 5.20 | 10.40 | 10.40 |
| | Pregelatinized starch | 25.00 | 25.00 | 25.00 |
| | Microcrystalline cellulose | 33.40 | 18.20 | 20.20 |
| | Crospovidone | 6.40 | 6.40 | 6.40 |
| | Hydroxypropyl cellulose | 2.00 | 2.00 | 2.00 |
| | Anhydrous dibasic calcium phosphate | 12.00 | 22.00 | |
| | Calcium carbonate | | | 20.00 |
| Further mixture | Crospovidone | 20.00 | 20.00 | 20.00 |
| | Magnesium stearate | 4.80 | 4.80 | 4.80 |
| Total weight | | 406.00 | 406.00 | 406.00 |

### Example 15 - A single-layered tablet formulated by preparing the granule of valsartan and amlodipine and mixing therewith second composition containing rosuvastatin

Using the same method as Example 1, a single-layered tablet having the ingredients and contents shown in Table 10 below was prepared.

**[Table 10]**

| Single-layered tablet | Ingredients | Example 15 |
|---|---|---|
| First granule | Valsartan | 160.00 |
| | Amlodipine besylate | 6.94 |
| | Microcrystalline cellulose | 90.66 |
| | Croscarmellose sodium | 16.00 |
| | Hydroxypropyl cellulose | 9.60 |
| | Alumina magnesium metasilicate | 16.00 |
| Second granule | Rosuvastatin calcium | 10.40 |
| | Pregelatinized starch | 25.00 |
| | Microcrystalline cellulose | 20.00 |
| | Crospovidone | 18.40 |
| | Anhydrous dibasic calcium phosphate | 18.20 |
| | Crospovidone | 10.00 |
| | Magnesium stearate | 4.80 |
| Total weight | | 406.00 |

### Example 16 - A double-layered tablet comprising the first layer containing the granule of valsartan and amlodipine and the second layer containing rosuvastatin granule

Using the same method as Example 5, a double-layered tablet having the ingredients and contents shown in Table 11 below was prepared.

**[Table 11]**

| Double-layered tablet | | Ingredients | Example 16 |
|---|---|---|---|
| First layer | First granule | Valsartan | 160.00 |
| | | Amlodipine besylate | 6.94 |
| | | Microcrystalline cellulose | 90.66 |
| | | Croscarmellose sodium | 16.00 |
| | | Polyvinyl pyrrolidone | 9.60 |
| | | Alumina magnesium metasilicate | 16.00 |
| | Further mixture | Crospovidone | 16.00 |
| | | Magnesium stearate | 2.40 |
| Second layer | Second granule | Rosuvastatin calcium | 10.40 |
| | | Pregelatinized starch | 25.00 |
| | | Microcrystalline cellulose | 20.00 |
| | | Crospovidone | 6.40 |
| | | Hydroxypropyl cellulose | 2.00 |
| | | Anhydrous dibasic calcium phosphate | 18.20 |
| | Further mixture | Crospovidone | 5.00 |
| | | Magnesium stearate | 1.40 |
| Total weight | | | 406.00 |

### Comparative Example 1 - A single-layered tablet formulated by preparing the granule of valsartan and amlodipine and mixing therewith the second composition containing rosuvastatin

Using the same method as Example 2, a single-layered tablet having the ingredients and contents shown in Table 12 below was prepared. In contrast to Example 2, a single-layered tablet was formulated by preparing the granule of rosuvastatin and amlodipine as the first composition and mixing therewith the valsartan granule as the second composition.

**[Table 12]**

| Comparative Example 1 | Ingredients | Content (mg) |
|---|---|---|
| First granule | Rosuvastatin calcium | 10.40 |
| | Amlodipine besylate | 6.94 |
| | Pregelatinized starch | 25.00 |
| | Microcrystalline cellulose | 20.00 |
| | Crospovidone | 6.40 |
| | Hydroxypropyl cellulose | 2.00 |
| | Anhydrous dibasic calcium phosphate | 18.20 |
| Second granule | Valsartan | 160.00 |
| | Microcrystalline cellulose | 90.66 |
| | Croscarmellose sodium | 16.00 |
| | Hydroxypropyl cellulose | 9.60 |
| | Alumina magnesium metasilicate | 16.00 |
| Further mixture | Crospovidone | 20.00 |
| | Magnesium stearate | 4.80 |
| Total weight | | 406.00 |

### Comparative Examples 2 and 3 - A single-layered tablet formulated by preparing the granule of valsartan and amlodipine and mixing therewith rosuvastatin granule, in which the first composition has different excipients from that of Examples

Using the same method as Example 2, a single-layered tablet having the ingredients and contents shown in Table 13 below was prepared. In contrast to Examples 7 to 10, mannitol and lactose were used as excipients of the first composition instead of alumina magnesium metasilicate and low-substituted hydroxypropyl cellulose.

**[Table 13]**

| | Ingredients | Comparative Example 2 | Comparative Example 3 |
|---|---|---|---|
| First granule | Valsartan | 160.00 | 160.00 |
| | Amlodipine besylate | 6.94 | 6.94 |
| | Microcrystalline cellulose | 90.66 | 90.66 |
| | Croscarmellose sodium | 16.00 | 16.00 |
| | Hydroxypropyl cellulose | 9.60 | 9.60 |
| | Mannitol | 16.00 | |
| | Lactose | | 16.00 |
| Second granule | Rosuvastatin calcium | 10.40 | 10.40 |
| | Pregelatinized starch | 25.00 | 25.00 |
| | Microcrystalline cellulose | 20.00 | 20.00 |
| | Crospovidone | 6.40 | 6.40 |
| | Hydroxypropyl cellulose | 2.00 | 2.00 |
| | Anhydrous dibasic calcium phosphate | 18.20 | 18.20 |
| Further mixture | Crospovidone | 20.00 | 20.00 |
| | Magnesium stearate | 4.80 | 4.80 |
| Total weight | | 406.00 | 406.00 |

### Comparative Examples 4 and 5 - A single-layered tablet preparing the granule of valsartan and amlodipine and mixing therewith rosuvastatin granule, in which the second composition has different excipients from that of Examples 13 to 15

Using the same method as Example 2, a single-layered tablet having the ingredients and contents shown in Table 14 below was prepared. In contrast to Examples 7, and 11 to 13, meglumine and magnesium oxide were used as excipients of the second composition instead of calcium carbonate and anhydrous dibasic calcium phosphate.

**[Table 14]**

| | Ingredients | Comparative Example 4 | Comparative Example 5 |
|---|---|---|---|
| First granule | Valsartan | 160.00 | 160.00 |
| | Amlodipine besylate | 6.94 | 6.94 |
| | Microcrystalline cellulose | 90.66 | 90.66 |
| | Croscarmellose sodium | 16.00 | 16.00 |
| | Hydroxypropyl cellulose | 9.60 | 9.60 |
| | Alumina magnesium metasilicate | 16.00 | 16.00 |
| Second granule | Rosuvastatin calcium | 10.40 | 10.40 |
| | Pregelatinized starch | 25.00 | 25.00 |
| | Microcrystalline cellulose | 20.00 | 20.00 |
| | Crospovidone | 6.40 | 6.40 |
| | Hydroxypropyl cellulose | 2.00 | 2.00 |
| | Meglumine | 18.20 | |
| | Magnesium oxide | | 18.20 |
| Further mixture | Crospovidone | 20.00 | 20.00 |
| | Magnesium stearate | 4.80 | 4.80 |
| Total weight | | 406.00 | 406.00 |

### Experimental Example 1. Stability test

The tablets of the Examples and the Comparative Examples were coated, and packed into bottles (including silica gel) and aluminum packages, and then stability test for the packaged tablets under severe storage conditions was carried out to check for chemical stability between the major ingredients and the excipients. Specifically, the tablets of each Example were packaged in Alu Alu, and then, each packaged vial was stored at severe conditions of 60°C/80% (relative humidity) for 4 weeks. Total impurity contents (%) were examined via HPLC.

The condition used for HPLC analysis is as follows:
Column: (250×4.6 mm, 5 µm) phenomenex kinetex Biphenyl
Detector: absorption photometer (237 nm)
Mobile phase: acetonitrile : water : trifluoroacetic acid mixed solution = 37:63:0.3
Flow rate: 1.0 mL/min

### Experimental Example 1-1. Stability test of a composition including valsartan, amlodipine, and rosuvastatin

As shown in Table 15 below, it was confirmed in Examples 1 to 3, which have valsartan and amlodipine as the first composition and rosuvastatin as the second composition, that the stability was effectively maintained as there was no significant difference in the total impurity contents.

**[Table 15]**

| Total impurity contents | | Initial | Al/Al | | Bottle | |
|---|---|---|---|---|---|---|
| | | | 2 weeks under severe conditions | 4 weeks under severe conditions | 2 weeks under severe conditions | 4 weeks under severe conditions |
| Example 1 | Am-imp | 0.049 | 0.205 | 0.193 | 0.072 | 0.083 |
| | Ro-imp | 0.194 | 1.043 | 1.142 | 1.005 | 1.156 |
| | Val-imp | 0.211 | 0.213 | 0.210 | 0.224 | 0.216 |
| Example 2 | Am-imp | 0.062 | 0.099 | 0.109 | 0.085 | 0.082 |
| | Ro-imp | 0.222 | 1.121 | 1.186 | 1.209 | 1.334 |
| | Val-imp | 0.211 | 0.211 | 0.217 | 0.216 | 0.224 |
| Example 3 | Am-imp | 0.206 | 0.206 | 0.116 | 0.111 | 0.107 |
| | Ro-imp | 0.278 | 1.519 | 1.519 | 1.209 | 1.381 |
| | Val-imp | 0.209 | 0.209 | 0.210 | 0.222 | 0.248 |

Additionally, stability of the formulations prepared in Examples 3, 4, and 6 under 2 weeks of severe conditions was tested. Purity of rosuvastatin among the major ingredients, which has relatively poor stability, was examined. Further, as the Al/Al packaging is less table compared to the vial packaging, the Al/Al packaging was examined.

**[Table 16]**

| Total impurity contents | | Initial | 2 weeks under severe conditions |
|---|---|---|---|
| Example 6 | Ro-imp | 1.3 | 2.0 |
| Example 3 | Ro-imp | 1.33 | 2.4 |
| Example 4 | Ro-imp | 1.06 | 2.87 |

Consequently, as shown in Table 16 above, the stability of the double-layered tablet of Example 6 and that of the single-layered tablets of Examples 3 and 4 were examined and found to have excellent stability.

Based on the result of Experimental Example 1-1, it was confirmed that when valsartan and amlodipine were included as the first composition and rosuvastatin was included as the second composition, interactions among the major ingredients or those between the major ingredients the excipients were minimized, thereby increasing stability.

### Experimental Example 1-2. Comparison of excipients affecting the stability of the major ingredients

A stability test of amlodipine besylate, valsartan, and rosuvastatin was conducted for the formulations prepared in Examples 11 to 13 and Comparative Examples 4 and 5. Specifically, in Examples 11 to 13, anhydrous dibasic calcium phosphate and calcium carbonate were used as excipients, and the ratio of each example was respectively 3%, 3%, and 5% based on the total weight. Comparative Examples 4 and 5 used meglumine and magnesium oxide as excipients, and ratios thereof were 4% and 4% based on the total weight. Stability of the coated and aluminum-packed formulations of Examples 11 to 13 and Comparative Examples 4 and 5 was tested under severe conditions for 2 weeks. Among the major ingredients, the purity of rosuvastatin with relatively poor stability was examined, as shown in Table 17 below.

**[Table 17]**

| Total impurity contents | | Initial | 2 weeks under severe conditions |
|---|---|---|---|
| Example 11 | Ro-imp | 0.8 | 1.3 |
| Example 12 | Ro-imp | 0.8 | 0.9 |
| Example 13 | Ro-imp | 0.5 | 0.8 |
| Example 4 | Ro-imp | 0.6 | 2.7 |
| Example 5 | Ro-imp | 0.2 | 0.3 |

As shown in Table 17 above, the stability or Examples 11 to 13, and that or Comparative Example 5 were examined and found to be excellent, whereas the stability of Comparative Example 4 was relatively poor. Therefore, based on the result of Experimental Example 1-2, it was confirmed that anhydrous dibasic calcium phosphate and calcium carbonate are the excipients which influence the stability of the major ingredients, especially rosuvastatin.

### Experimental Example 2. Dissolution test

In order to compare the dissolution rates of the combination formulation according to the present disclosure and the formulation for co-administering the control drugs of Exforge Tab. 5/160 mg and Crestor Tab. 10 mg, or in order to confirm that the combination formulation according to the present disclosure has a superior dissolution rate, a dissolution test was performed using the combination formulation prepared in the Examples.

Specifically, the dissolution rates were tested at 37°C in 900 mL of dissolution media at 50 rpm according to the dissolution method (Method II) disclosed in the USP. Dissolution media samples were collected at each scheduled time and analyzed via HPLC.

The condition used for HPLC analysis is as follows:
Column: (250×4.6 mm, 5 µm) phenomenex kinetex Biphenyl
Detector: absorption photometer (237 nm)
Mobile phase: acetonitrile : water : trifluoroacetic acid mixed solution = 45:55:0.2
Flow rate: 1.0 mL/min
Temperature of column: 40°C

### Experimental Example 2-1. Comparison of dissolution rate of single-layered tablets of Examples 3 and 4 and the control

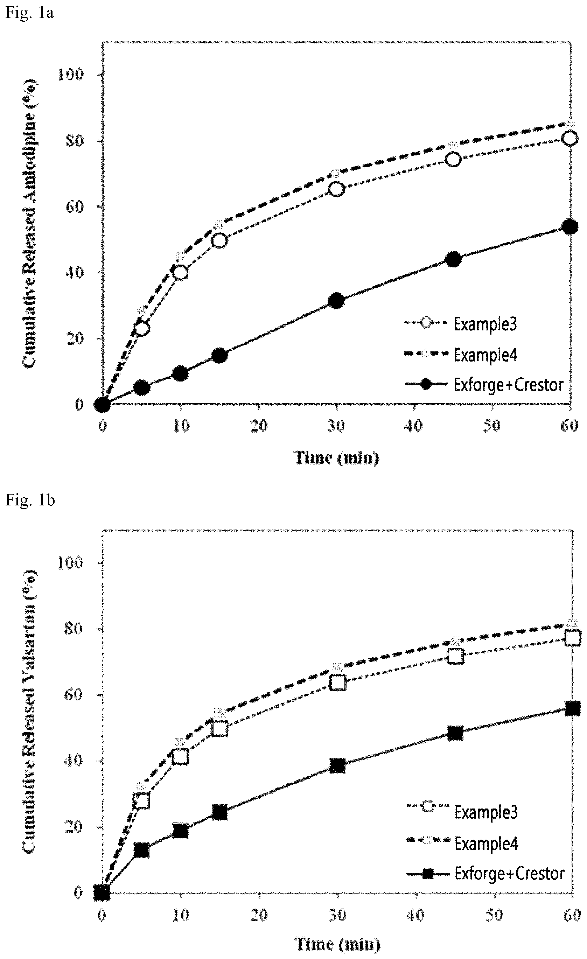

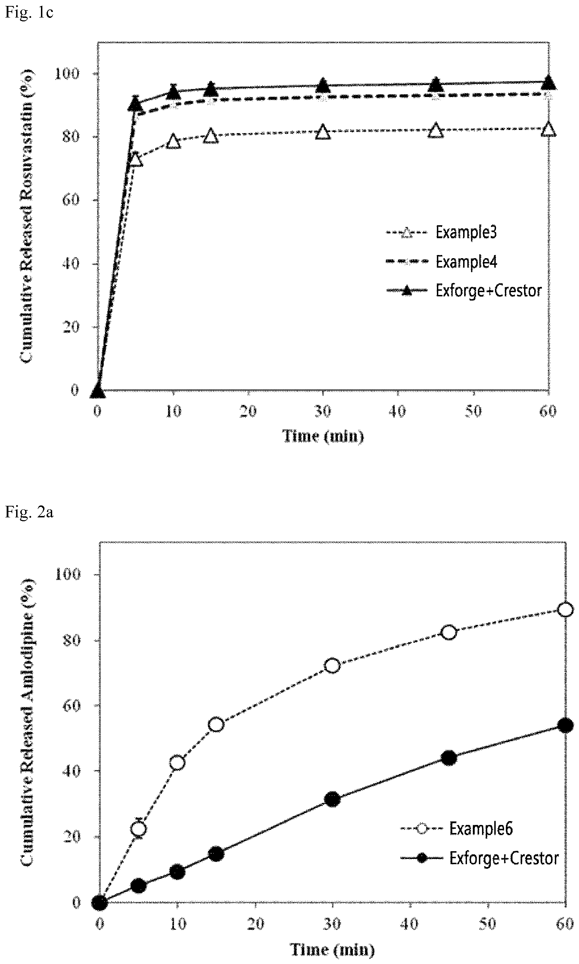

A dissolution rate test of amlodipine besylate, valsartan, and rosuvastatin was conducted for the formulations prepared in Examples 3 and 4 and the control formulations. The dissolution rate test result is shown in Table 18 below and Figs. 1a to 1c.

**[Table 18]**

| | Valsartan | | | Amlodipine | | | Rosuvastatin | | |
|---|---|---|---|---|---|---|---|---|---|
| Dissolution time (min) | Example 3 | Example 4 | Control formulation( Exforge + Crestor) | Example 3 | Example 4 | Control formulation( Exforge + Crestor) | Example 3 | Example 4 | Control formulation( Exforge + Crestor) |
| 5 | 27.9±1.6 | 32.1±0.4 | 13.1±0.8 | 23.3±1.8 | 28.1±1 | 5.2±0.9 | 73±2 | 86.8±1.3 | 90.5±2.3 |
| 10 | 41.4±0.6 | 45.6±0.4 | 18.9±1.1 | 40±1.1 | 45.2±1 | 9.5±1 | 78.9±0.5 | 90.3±1.1 | 94.3±2.1 |
| 15 | 49.8±0.4 | 54.4±0.7 | 24.5±1.2 | 49.9±0.7 | 54.9±1 | 15±1.4 | 80.5±0.4 | 91.7±1.1 | 95.3±1.5 |
| 30 | 63.8±0 | 68.2±0.9 | 38.6±1.4 | 65.4±0.1 | 70.3±1.5 | 31.5±2.1 | 81.8±0.2 | 92.5±0.9 | 96.3±1.4 |
| 45 | 71.8±0.1 | 76.3±1.1 | 48.5±1.2 | 74.5±0.1 | 78.8±1 | 44.2±1.9 | 82.2±0.1 | 93.1±0.7 | 96.6±1.5 |
| 60 | 77.4±0.3 | 81.6±1.2 | 56.1±1.3 | 80.9±0 | 85.3±1.6 | 54±2.2 | 82.7±0 | 93.5±0.7 | 97.4±1.3 |

Consequently, as shown in Table 18 above, it can be understood from Examples 3 and 4 that the preferable pharmacokinetic profiles of valsartan and amlodipine, which are ingredients to be promptly released *in vivo,* were well achieved (an increase in dissolution rates compared to the control formulation resulted in excellent Dog PK). Additionally, in the case of co-administering rosuvastatin with the control formulation, a similar dissolution pattern was observed.

### Experimental Example 2-2. Comparison in dissolution rates of the double-layered tablet of Example 6 and the control formulation

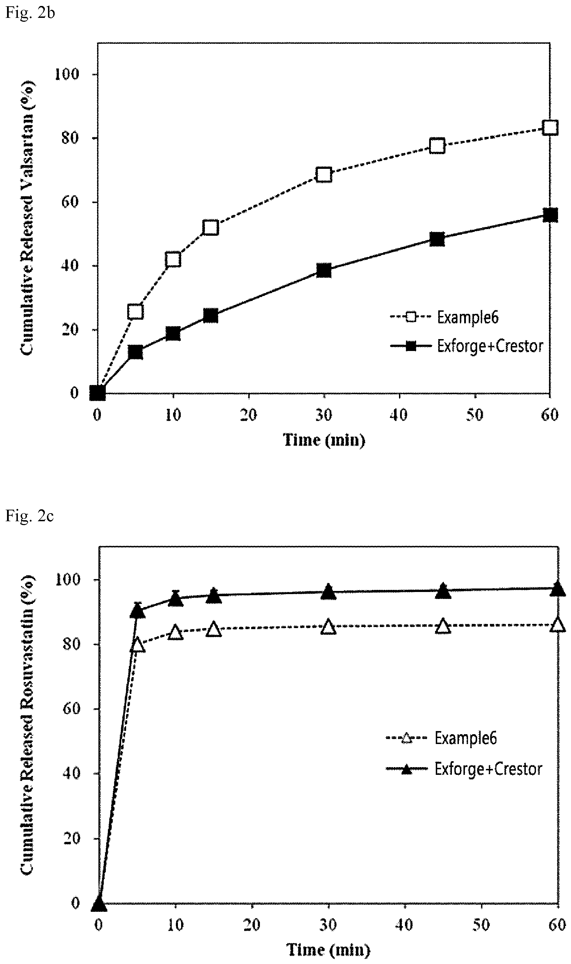

A dissolution rate test of amlodipine besylate, valsartan, and rosuvastatin was conducted for the formulations prepared in Example 8 and the control formulations. The dissolution rate test result is shown in Table 19 below and Figs. 2a to 2c.

**[Table 19]**

| | Valsartan | | Amlodipine | | Rosuvastatin | |
|---|---|---|---|---|---|---|
| Dissolution time (min) | Example 6 | Control formulation (Exforge + Crestor) | Example 6 | Control formulation( Exforge + Crestor) | Example 6 | Control formulation( Exforge + Crestor) |
| 5 | 25.8±1.5 | 13.1±0.8 | 22.5±3 | 5.2±0.9 | 80±0.1 | 90.5±2.3 |
| 10 | 42±0.1 | 18.9±1.1 | 42.6±0.5 | 9.5±1 | 83.8±0.4 | 94.3±2.1 |
| 15 | 52.2±0 | 24.5±1.2 | 54.3±0.6 | 15±1.4 | 84.8±0.7 | 95.3±1.5 |
| 30 | 68.7±0.3 | 38.6±1.4 | 72.3±0 | 31.5±2.1 | 85.6±0.8 | 96.3±1.4 |
| 45 | 77.6±0.5 | 48.5±1.2 | 82.6±1.1 | 44.2±1.9 | 85.8±0.8 | 96.6±1.5 |
| 60 | 83.3±0.4 | 56.1±1.3 | 89.5±1 | 54±2.2 | 86.2±0.9 | 97.4±1.3 |

Consequently, as shown in Table 19 above, it can be understood from Example 6 that the preferable pharmacokinetic profiles of valsartan and amlodipine, which are ingredients to be promptly released *in vivo,* were well achieved (an increase in dissolution rates compared to the control formulation resulted in excellent Dog PK). Meanwhile, in the case of co-administering rosuvastatin with the control formulation, a similar dissolution pattern was observed.

### Experimental Example 2-3. Comparison in dissolution rates according to changes in the granule compositions of the major ingredients

A dissolution rate test of amlodipine besylate, valsartan, and rosuvastatin was conducted for the formulations prepared in Example 7 and Comparative Example 1. The dissolution rate test results are shown in Table 20 below.

**[Table 20]**

| | Valsartan | | Amlodipine | | Rosuvastatin | |
|---|---|---|---|---|---|---|
| Dissolution time (min) | Example 7 | Comparative Example 1 | Example 7 | Comparative Example 1 | Example 7 | Comparative Example 1 |
| 5 | 24±1.2 | 10.2±0.1 | 24.4±1.5 | 29.9±1.5 | 72.2±0.3 | 55.3±0.8 |
| 10 | 36.8±0.7 | 16.4±0.1 | 41.7±0.4 | 53.5±1.6 | 84.3±0.5 | 73.3±1.4 |
| 15 | 46±0.9 | 20.3±0.1 | 53.7±1.6 | 64.7±0.5 | 88.9±0.2 | 81.1±2 |
| 30 | 63.2±0.6 | 27±0.1 | 73.3±0.8 | 78.6±1.4 | 94±0.1 | 90.1±2.4 |
| 45 | 72.5±0.4 | 30.8±0.2 | 82.7±1.3 | 84.1±1.6 | 95.7±0.2 | 93.1±2.5 |
| 60 | 78.7±0.3 | 33.4±0.3 | 88.4±0.9 | 87.1±2.6 | 96.8±0.3 | 94.2±2.9 |

Consequently, as shown in Table 20 above, compared to Example 7, Comparative Example 1 showed that valsartan had a lower dissolution rate and rosuvastatin had a lower dissolution rate during early dissolution. Additionally, the amlodipine/rosuvastatin granule turned light green overall. From such results, it could be confirmed that separating valsartan and amlodipine as the first composition and rosuvastatin as the second composition would result in an excellent dissolution rate.

Further, in the case of Example 7, the dissolution rate of rosuvastatin was very similar to that of rosuvastatin contained in the combination formulation of the control formulations disclosed in Experimental Examples 2-1 and 2-2, thereby confirming the most preferable single-layered tablet.

### Experimental Example 2-4. Comparison of dissolution rate according to the change in major excipient

A dissolution rate test of amlodipine besylate, valsartan, and rosuvastatin was conducted for the formulations prepared in Examples 8 to 10, 14, and Comparative Examples 2 and 3. Specifically, 80 mg of valsartan was used in Example 8 instead of 160 mg, and 10 mg of amlodipine was used in Example 9 instead of 5 mg. Example 10 used low-substituted hydroxypropyl cellulose instead of alumina magnesium metasilicate. Additionally, in Example 14, 20 mg of rosuvastatin and 10 mg of amlodipine were used.

Meanwhile, Examples 8 to 10 used alumina magnesium metasilicate and low-substituted hydroxypropyl cellulose as excipients, and the rates of the excipients were respectively 3%, 6%, and 15% with respect to the total weight. Comparative Examples 2 and 3 used mannitol and lactose as excipients, and the rates of the excipients were respectively 4% and 4% with respect to the total weight.

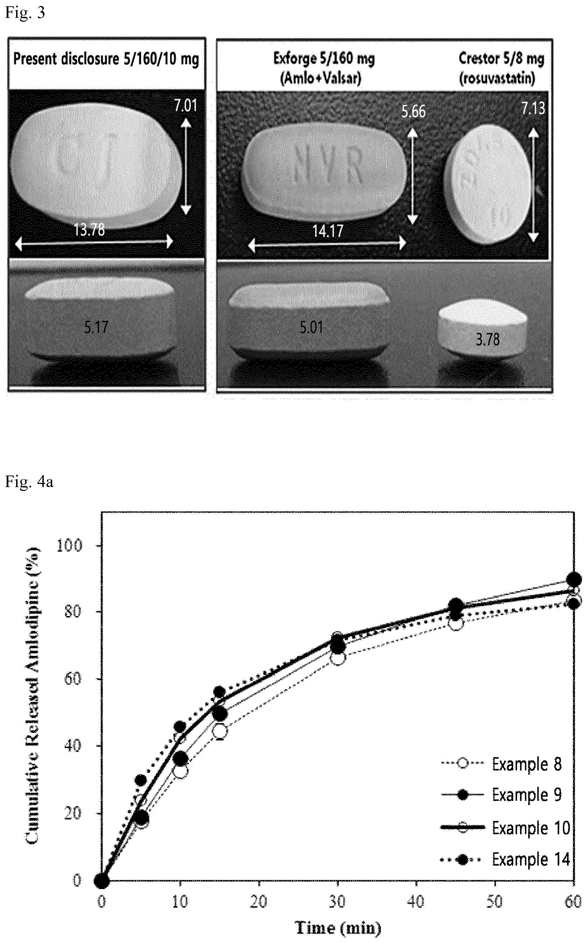

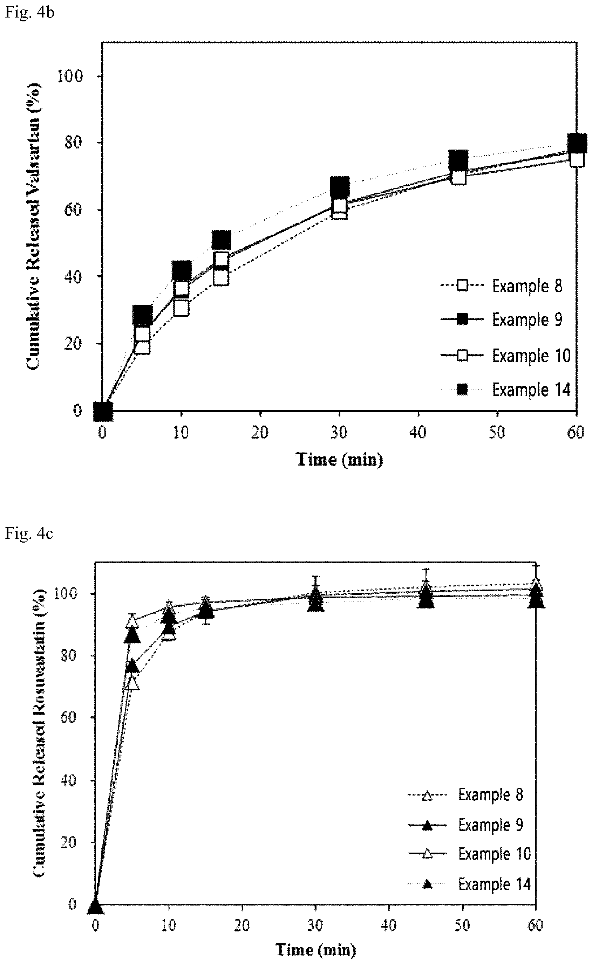

The dissolution test results are as shown in Tables 21 to 23 below and Figs. 4a to 4c.

**[Table 21]**

| | Valsartan | | | | | |
|---|---|---|---|---|---|---|
| Dissolution time (min) | Example 8 | Example 9 | Example 10 | Example 14 | Comparative Example 2 | Comparative Example 3 |
| 5 | 19.3±0.4 | 23±0.2 | 22±0.4 | 28.8±0.9 | 5.3±0.5 | 12±0 |
| 10 | 30.8±0.7 | 35.9±1 | 31.6±0.4 | 41.9±1.6 | 11.6±0.2 | 19.9±0.6 |
| 15 | 39.9±0.9 | 44.9±1.6 | 38.9±0.2 | 51.2±2.2 | 16.4±0.5 | 25.8±0.8 |
| 30 | 59.5±0.1 | 62±2.7 | 52±0.7 | 67±2.1 | 26.6±1.6 | 37.1±1 |
| 45 | 70.5±0 | 71.3±2.7 | 60.3±0.5 | 75.3±1.8 | 34.5±2.1 | 45.5±1.6 |
| 60 | 78±0 | 77.6±2.5 | 67.2±0.9 | 80±1.9 | 40.9±2.4 | 51.9±1.8 |

**[Table 22]**

| | Amlodipine | | | | | |
|---|---|---|---|---|---|---|
| Dissolution time (min) | Example 8 | Example 9 | Example 10 | Example 14 | Comparative Example 2 | Comparative Example 3 |
| 5 | 17.8±0.9 | 19.1±1.1 | 34.3±1 | 29.8±1.49 | 4.5±0 | 8.9±0.3 |
| 10 | 32.7±1.6 | 36.2±1.1 | 50.8±0 | 45.5±2.4 | 8.8±0 | 17.4±0 |
| 15 | 44.4±2.5 | 49.7±1 | 61.2±1.1 | 56.1±3.1 | 12.5±0.3 | 18.5±0.8 |
| 30 | 66.5±0.7 | 69.7±0.6 | 75.4±0.4 | 71.7±2.7 | 21.2±1.6 | 36.1±0.9 |
| 45 | 77±0.8 | 82.2±0.6 | 83.2±0.5 | 78.9±2.4 | 28.7±2 | 45.5±1.5 |
| 60 | 83.5±0.4 | 89.8±0.6 | 87.5±0.7 | 82.5±2.2 | 34.9±2.5 | 52±2.1 |

**[Table 23]**

| | Rosuvastatin | | | | | |
|---|---|---|---|---|---|---|
| Dissolution time (min) | Example 8 | Example 9 | Example 10 | Example 14 | Comparative Example 2 | Comparative Example 3 |
| 5 | 71.5±1.1 | 77.2±1 | 81.6±07 | 87.3±1 | 38.3±3.25 | 77.3±1.1 |
| 10 | 87.7±2.8 | 89.4±1.5 | 88.7±0.2 | 93.6±1 | 71.3±1.8 | 91.4±4.7 |
| 15 | 94.2±4 | 94.5±2 | 91.1±0.7 | 95.5±1 | 83.4±2.5 | 95.7±4.8 |
| 30 | 100.4±5 | 99.5±3.1 | 93.4±1.3 | 97.2±0.8 | 94.3±3.7 | 99.6±4.9 |
| 45 | 102.4±5 | 100.8±3 | 94.2±1 | 98.3±0.8 | 98.6±3.3 | 101.3±5 |
| 60 | 103.4±5.5 | 101.4±3.2 | 94±1.2 | 98.6±0.9 | 101.2±3.3 | 101.6±4.9 |

Consequently, as shown in Tables 21 to 23 above, all major ingredients showed high dissolution rates in Examples 8 to 10, and 14. However, in Comparative Examples 2 and 3 valsartan and amlodipine showed lower dissolution rates compared to the above Examples, and in Comparative Example 3 rosuvastatin showed a lower dissolution rate during the early dissolution.

Accordingly, it was confirmed that alumina magnesium metasilicate and low-substituted hydroxypropyl cellulose are major excipients that influence the dissolution rates of the major ingredients.

Additionally, comparative dissolution tests for amlodipine besylate, valsartan, and rosuvastatin were conducted on the formulations prepared in Examples 11 to 13, and Comparative Example 5. Specifically, Examples 11 to 13 used anhydrous dibasic calcium phosphate or calcium carbonate as an excipient, whereas Comparative Example 5 used magnesium oxide. The test results are shown as Tables 24 to 26 below.

**[Table 24]**

| | Valsartan | | | |
|---|---|---|---|---|
| Dissolution time (min) | Example 11 | Example 12 | Example 13 | Comparative Example 5 |
| 5 | 23.2±0.5 | 24.6±1.1 | 35.1±0.5 | 48.6±0.7 |
| 10 | 36.6±0.7 | 38.6±1.2 | 50.5±0.4 | 66.1±0.1 |
| 15 | 45.5±0.8 | 48.2±1.2 | 59.1±0.3 | 75.7±0.1 |
| 30 | 61.4±0.6 | 64.3±0.5 | 72.6±0.8 | 88.5±0.4 |
| 45 | 70±0.8 | 72.9±0.9 | 79.5±1.1 | 93.9±0.2 |
| 60 | 75.3±0.5 | 78.1±1 | 83.8±1.1 | 97±0.5 |

**[Table 25]**

| | Amlodipine | | | |
|---|---|---|---|---|
| Dissolution time (min) | Example 11 | Example 12 | Example 13 | Comparative Example 5 |
| 5 | 23.7±0.6 | 24.2±1.9 | 34.1±0.4 | 49.4±1 |
| 10 | 42.2±1.1 | 44.2±1.9 | 55.6±0.8 | 72.9±0.1 |
| 15 | 53.5±1.6 | 57.2±2 | 66.6±0.5 | 84.9±0.5 |
| 30 | 72.4±0.5 | 75.8±0.3 | 80.6±1.7 | 94.9±0.4 |
| 45 | 81.2±0.2 | 84.5±0.4 | 86.9±2.2 | 98.4±0.7 |
| 60 | 86.4±0.4 | 89.4±0.7 | 90.9±2.2 | 99.6±1.1 |

**[Table 26]**

| | Rosuvastatin | | | |
|---|---|---|---|---|
| Dissolution time (min) | Example 11 | Example 12 | Example 13 | Comparative Example 5 |
| 5 | 91.2±2.3 | 81.6±1.5 | 85.2±0.7 | 93.7±2.1 |
| 10 | 95.8±1.7 | 90.8±2 | 93.7±2 | 99.7±2.1 |
| 15 | 97.4±1.3 | 94.1±2.4 | 95.7±2.4 | 101.4±2 |
| 30 | 98.9±0.8 | 97.1±2.7 | 97.8±2.1 | 102.2±2.1 |
| 45 | 99.4±1.1 | 98.1±2.4 | 98.4±2.2 | 102.4±1.8 |
| 60 | 99.7?0.7 | 98.9±2 | 98.9±2.3 | 102.8±2.1 |

As shown above, the result shows that in comparison of Comparative Example 5 with the Examples, amlodipine and valsartan showed higher dissolution rates in Example 5 whereas rosuvastatin showed similar dissolution rates in Example 5. When dissolution rates are higher than in the Examples, which is the case with Comparative Example 5, , it is highly likely that a bioavailability test would result in non-equivalence due to deviation from the upper limits. Accordingly, as confirmed in Experimental Examples 1-2, Comparative Example 5 has reasonable stability, but not a desirable dissolution rate.

### Experimental Example 2-5. Comparison of dissolution rate of single-layered tablet and double-layered tablet

A comparative dissolution test for amlodipine besylate, valsartan, and rosuvastatin was conducted on the formulations prepared in Examples 15 and 16. Specifically, a single-layered tablet in which the granule of amlodipine and valsartan is further mixed with rosuvastatin in Example 15, and a double-layered tablet which has a first layer of amlodipine and valsartan and a second layer of rosuvastatin was prepared in Example 16. The result of the dissolution experiment thereof is shown in Tables 27 to 29 below.

**[Table 27]**

| | Valsartan | |
|---|---|---|
| Dissolution time (min) | Example 15 | Example 16 |
| 5 | 27.5±0.5 | 23.6±0.9 |
| 10 | 43±0.2 | 38.4±0.3 |
| 15 | 53.6±0.1 | 48.4±0.1 |
| 30 | 70.3±0.2 | 64.7±0.2 |
| 45 | 78.3±0.1 | 73.6±0.2 |
| 60 | 83.1±0.1 | 78.8±0.2 |

**[Table 28]**

| | Amlodipine | |
|---|---|---|
| Dissolution time (min) | Example 15 | Example 16 |
| 5 | 28.9±1.7 | 27.8±1.2 |
| 10 | 50.5±0.9 | 47.8±0.5 |
| 15 | 65.3±0.3 | 61.2±0.5 |
| 30 | 83.9±0.3 | 79±0 |
| 45 | 91.4±0.4 | 87.6±0 |
| 60 | 94.9±0.2 | 92.3±0.2 |

**[Table 29]**

| | Rosuvastatin | |
|---|---|---|
| Dissolution time (min) | Example 15 | Example 16 |
| 5 | 89.2±0.7 | 76±3.8 |
| 10 | 94.6±0.8 | 91.2±6 |
| 15 | 96.3±1 | 95.9±6.4 |
| 30 | 98.1±1.6 | 99.9±6.2 |
| 45 | 98.5±1.7 | 101.8±6 |
| 60 | 98.7±2 | 102.3±5.2 |

As a result, as shown in the table above, Examples 15 and 16 had similar dissolution rates of amlodipine, valsartan, and rosuvastatin as in the other Examples.

In light of the above results, it was confirmed that according to the present disclosure, single-layered tablets showed stability and a dissolution rate equivalent to those of double-layered tablets. Therefore, it is advantageous in that a single-layered tablet is provided with a simple preparation process and an inexpensive process cost. Additionally, according to the present disclosure, it is advantageous in that not only is stability guaranteed without interactions between the major ingredients, but also the weight and size are less than those of the combination of the control formulations, Exforge Tab. (340 mg) and Crestor Tab. (155 mg) (see Fig. 3).

## Claims

1. A pharmaceutical composition, comprising
a first composition which comprises amlodipine or a pharmaceutically acceptable salt thereof and valsartan or a pharmaceutically acceptable salt thereof; and
a second composition which comprises rosuvastatin or a pharmaceutically acceptable salt thereof,
wherein the first composition further comprises magnesium aluminosilicate, low-substituted hydroxypropyl cellulose, or a mixture thereof; and
wherein the second composition further comprises anhydrous dibasic calcium phosphate, calcium carbonate, or a mixture thereof.

2. The pharmaceutical composition of claim 1, wherein the first composition is in the form of a granule; and the pharmaceutical composition is a single-layered tablet wherein the first and the second compositions are mixed and compressed.

3. The pharmaceutical composition of claim 1, wherein the first and the second compositions are in the form of granules; and the pharmaceutical composition is a double-layered tablet having the first composition as a first layer and the second composition as a second layer.

4. The pharmaceutical composition of claim 1 , wherein the pharmaceutical composition comprises 5 mg to 10 mg of amlodipine, 80 mg to 160 mg of valsartan, or 5 mg to 20 mg of rosuvastatin per unit dosage form.

5. The pharmaceutical composition of claim 1, wherein the magnesium aluminosilicate, low-substituted hydroxypropyl cellulose, or mixture thereof is comprised at 3wt% to 15 wt% based on the total weight of the pharmaceutical composition.

6. The pharmaceutical composition of claim 1, wherein the anhydrous dibasic calcium phosphate, calcium carbonate, or mixture thereof is comprised at 3 wt% to 5 wt% based on the total weight of the pharmaceutical composition.

## Patentansprüche

1. Pharmazeutische Zusammensetzung, umfassend:
eine erste Zusammensetzung, die Amlodipin oder ein pharmazeutisch unbedenkliches Salz davon und Valsartan oder ein pharmazeutisch unbedenkliches Salz davon umfasst, und
eine zweite Zusammensetzung, die Rosuvastatin oder ein pharmazeutisch unbedenkliches Salz davon umfasst,
wobei die erste Zusammensetzung weiterhin Magnesiumaluminosilicat, Hydroxypropylcellulose mit niedrigem Substitutionsgrad oder eine Mischung davon umfasst und
wobei die zweite Zusammensetzung weiterhin wasserfreies Calciumhydrogenphosphat, Calciumcarbonat oder eine Mischung davon umfasst.

2. Pharmazeutische Zusammensetzung nach Anspruch 1, wobei die erste Zusammensetzung in Form eines Granulats vorliegt und es sich bei der pharmazeutischen Zusammensetzung um eine Einschichttablette handelt, in der die erste und die zweite Zusammensetzung gemischt und komprimiert sind.

3. Pharmazeutische Zusammensetzung nach Anspruch 1, wobei die erste und die zweite Zusammensetzung in Form von Granulaten vorliegen und es sich bei der pharmazeutischen Zusammensetzung um eine Zweischichttablette mit der ersten Zusammensetzung als eine erste Schicht und der zweiten Zusammensetzung als eine zweite Schicht handelt.

4. Pharmazeutische Zusammensetzung nach Anspruch 1, wobei die pharmazeutische Zusammensetzung 5 mg bis 10 mg Amlodipin, 80 mg bis 160 mg Valsartan oder 5 mg bis 20 mg Rosuvastatin pro Einheitsdosisform umfasst.

5. Pharmazeutische Zusammensetzung nach Anspruch 1, wobei das Magnesiumaluminosilicat, die Hydroxypropylcellulose mit niedrigem Substitutionsgrad oder die Mischung davon 3 Gew.-% bis 15 Gew.-%, bezogen auf das Gesamtgewicht der pharmazeutischen Zusammensetzung, ausmacht.

6. Pharmazeutische Zusammensetzung nach Anspruch 1, wobei das wasserfreie Calciumhydrogenphosphat, Calciumcarbonat oder die Mischung davon 3 Gew.-% bis 5 Gew.-%, bezogen auf das Gesamtgewicht der pharmazeutischen Zusammensetzung, ausmacht.

## Revendications

1. Composition pharmaceutique, comprenant
une première composition qui comprend de l'amlodipine ou un sel pharmaceutiquement acceptable correspondant et du valsartan ou un sel pharmaceutiquement acceptable correspondant ; et
une deuxième composition qui comprend de la rosuvastatine ou un sel pharmaceutiquement acceptable correspondant,
la première composition comprenant en outre de l'aluminosilicate de magnésium, une hydroxypropylcellulose faiblement substituée, ou un mélange correspondant ; et
la deuxième composition comprenant en outre du phosphate de calcium dibasique anhydre, du carbonate de calcium, ou un mélange correspondant.

2. Composition pharmaceutique selon la revendication 1, la première composition étant sous la forme d'un granule ; et la composition pharmaceutique étant un comprimé à une seule couche, la première et la deuxième compositions étant mélangées et comprimées.

3. Composition pharmaceutique selon la revendication 1, la première et la deuxième compositions étant sous la forme de granules ; et la composition pharmaceutique étant un comprimé à double couche possédant la première composition comme une première couche et la deuxième composition comme une deuxième couche.

4. Composition pharmaceutique selon la revendication 1, la composition pharmaceutique comprenant 5 mg à 10 mg d'amlodipine, 80 mg à 160 mg de valsartan, ou 5 mg à 20 mg de rosuvastatine par forme de dosage unitaire.

5. Composition pharmaceutique selon la revendication 1, l'aluminosilicate de magnésium, l'hydroxypropylcellulose faiblement substituée, ou un mélange correspondant représentant 3 % en poids à 15 % en poids sur la base du poids total de la composition pharmaceutique.

6. Composition pharmaceutique selon la revendication 1, le phosphate de calcium dibasique anhydre, le carbonate de calcium, ou un mélange correspondant représentant 3 % en poids à 5 % en poids sur la base du poids total de la composition pharmaceutique.
